(19) Europäisches Patentamt

European Patent Office

Office européen des brevets

(11) **EP 0 618 956 B1**

(12) **EUROPEAN PATENT SPECIFICATION**

(45) Date of publication and mention
of the grant of the patent:
**11.07.2001 Bulletin 2001/28**

(51) Int Cl.[7]: **C10M 171/00**, C07C 67/313,
C07C 69/34

(21) Application number: **92925343.3**

(22) Date of filing: **17.11.1992**

(86) International application number:
**PCT/US92/10013**

(87) International publication number:
**WO 93/13188 (08.07.1993 Gazette 1993/16)**

(54) **POLYFUNCTIONAL MICHAEL ADDITION PRODUCTS AND REFRIGERATION COMPOSITIONS CONTAINING SUCH PRODUCTS**

POLYFUNKTIONELLE MICHAEL ADDITIONSPRODUKTE UND DIESE ENTHALTENDE KÜHLZUSAMMENSETZUNGEN

PRODUITS D'ADDITION MICHAEL POLYFONCTIONNELS ET COMPOSITIONS DE REFRIGERATION CONTENANT LESDITS PRODUITS

(84) Designated Contracting States:
**DE FR GB IT**

(30) Priority: **23.12.1991 US 812398**
**21.09.1992 US 947629**
**21.09.1992 US 947628**

(43) Date of publication of application:
**12.10.1994 Bulletin 1994/41**

(73) Proprietor: **ALBEMARLE CORPORATION**
**Baton Rouge Louisiana 70801 (US)**

(72) Inventor: **SABAHI, Mahmood**
**Baton Rouge, LA 70810 (US)**

(74) Representative: **Sandmair, Kurt, Dr. Dr.**
**Patentanwälte**
**Schwabe, Sandmair, Marx**
**Stuntzstrasse 16**
**81677 München (DE)**

(56) References cited:
**EP-A- 0 068 887**      **EP-A- 0 164 546**
**EP-A- 0 421 298**      **EP-A- 0 450 875**
**GB-A- 874 113**

• **DATABASE WPIL Week 8524, Derwent**
  **Publications Ltd., London, GB; AN 85-143099**
• **DATABASE WPIL Week 8426, Derwent**
  **Publications Ltd., London, GB; AN 84-161015**

## Description

Field of Invention

The invention relates to Michael addition products and to refrigeration compositions containing such products as lubricants.

Background

The Michael reaction is a known process wherein a Michael acceptor (such as an $\alpha,\beta$-ethylenically-unsaturated aldehyde, ester, nitrite, ketone, sulfone, or sulfoxide) is reacted with a Michael donor (such as a dialkyl malonate) to elongate a carbon chain. U.S. Patent 2,396,626 (Wiest et al.) teaches that products useful as plasticizers or solvents can be obtained by reacting two molecules of acrylonitrile, an alkyl acrylate, or an acrylamide with a molecule of a donor, such as an ester, amide, or nitrile of malonic acid, phenylacetic acid, cyanoacetic acid, or acetoacetic acid. However, as indicated in Skarzewski, "The Michael Reaction of Methanetricarboxylic Esters. A Simple Method for Two-Carbon Chain Elongation," *Synthesis*, December 1990, pp. 1125-1127, it has usually been considered undesirable to add a donor molecule to more than one acceptor molecule in such a reaction.

Summary of Invention

It has now been found that a Michael reaction can be conducted so as to form products containing 1-30 acceptor moieties/donor moiety, that oils thus produced have utility as lubricants (especially refrigeration lubricants), and that other novel products containing at least three acceptor moieties/donor moiety are also useful materials.

Thus, the invention resides in (1) compositions according to claims 11 to 13 comprising a refrigerant and, as a refrigeration lubricant, at least one oil of (2) a novel mixture of compounds according to claims 1 to 7, and (3) a processes according to claims 8 to 10 for preparing the novel compounds.

Detailed Description

The Michael donors employed in preparing the novel products are Z'-CH(E)(E") compounds wherein Z' is hydrogen or an alkyl or cycloalkyl group of up to 10 carbons, E" is hydrogen or an electron withdrawing group, and E is an electron withdrawing group — the electron withdrawing group or groups being any of those mentioned above but preferably being -COOR, -C(O)R', and/or -CN groups in which R and R' are alkyl or cycloalkyl groups of up to 10 carbons, preferably methyl or ethyl. The most preferred Michael donors are the dimethyl and diethyl malonates; the methyl and ethyl cyanoacetates, chloroacetates, acetoacetates, and propionylacetates; malononitrile; acetonitrile; acetylacetone; and dipropionylmethane.

The Michael acceptors are ordinarily CTT'=CT"G compounds in which T, T, and T" are independently selected from hydrogen, G', and organic groups (e.g., alkyl, alkoxyalkyl, alkylthioalkyl, cycloalkyl, dialkylaminocycloalkyl, aryl, haloaryl, alkoxyaryl, aralkyl, and alkaryl groups) of up to 20 carbons; and G and G' are electron withdrawing groups — the electron withdrawing group or groups being any of those mentioned above but preferably being -COOR, -C(O)R', and/or -CN groups in which R and R' are alkyl or cycloalkyl groups of up to 20 carbons.

Of these preferred compounds, the Michael acceptors which are apt to be most preferred are (A) those in which T, T, and T" are hydrogen and G is a -CN, -COOR, or -C(O)R' group wherein R and R' are methyl or ethyl and (B) the corresponding compounds in which one or two of the hydrogens represented by T, T', and T' is replaced with a G' electron withdrawing group which may be the same as G or a different group selected from -CN, -COOR, and C(O)R'.

The especially preferred Michael acceptors are the methyl and ethyl acrylates, acrylonitrile, dicyanoethylene, tricyanoethylene, methyl vinyl ketone, and ethyl vinyl ketone.

The reaction between the Michael donor and Michael acceptor may sometimes be conducted by a process such as that of Wiest et al., e.g., when the objective is to obtain oily Michael products containing two acceptor moieties/donor moiety. However, it is generally preferred to react the donor and acceptor in the presence of a basic compound and a phase transfer catalyst at a suitable temperature, usually a temperature of 0-150°C, preferably 20-80°C, and most preferably 40-60°C.

The basic compound, which serves to initiate the reaction, may be any other suitable base; but it is preferably an alkali or alkaline earth metal hydroxide, alkoxide, amide, or carbonate, more preferably a sodium or potassium hydroxide, alkoxide, amide, or carbonate, and most preferably potassium carbonate. Although it may be used in any amount sufficient to initiate the reaction, its concentration is usually 1-50%, preferably 3-30%, and most preferably 5-10%, based on the weight of the Michael donor.

The phase transfer catalyst employed in the process may be any such catalyst having sufficient catalytic activity to permit the addition of the desired number of Michael acceptor molecules to the Michael donor at a desired rate. Such catalysts include common phase transfer catalysts such as aluminum oxide, potassium fluoride, and mixtures thereof. However, the preferred catalysts are ordinarily alkylammonium salts such as tetraalkylammonium chlorides, bromides, fluorides, iodides, sulfates, hydrogen sulfates, carbonates, and phosphates in which the alkyl groups contain 1-20 carbons — salts which are frequently used as phase transfer catalysts. The phase transfer catalyst is used in a catalytic amount, typically an amount such as to provide 0.1-1 mol of catalyst per mol of Michael donor.

Although the Michael reaction of the invention is usually conducted in the absence of a solvent, it may sometimes be desirable to increase the efficiency of the phase transfer reaction by utilizing a solvent. The solvent, when used, should be a non-nucleophilic substance, e.g., a hydrocarbon, which will maintain the reactants in solution during the reaction but permit easy separation of the products from the reaction mixture. Such solvents include, e.g., toluene, xylene, other alkylbenzenes, hexane, and other saturated hydrocarbons.

The reaction is effected by combining the reactants, initiator, and catalyst, optionally in the presence of a solvent, and maintaining contact between the reactants at the selected reaction temperature until the desired degree of reaction has been effected. It is usually preferred to make the Michael acceptor the last of the ingredients to be charged to the reaction vessel in order to achieve better control of the reaction temperature and hence improved direction of the reaction to the formation of a desired product.

In the Michael reaction of the invention, the type of product formed is determined largely by the acceptor/ donor ratio in the reaction mixture — higher ratios leading to the formation of products containing more acceptor moieties per molecule and thus having higher molecular weights. Since the reaction normally leads to the formation of a mixture of products containing different numbers of acceptor moieties per molecule, it permits the production of some molecules containing more acceptor moieties than the number that would theoretically be provided by the amount of acceptor employed in the reaction mixture. However, it is necessary for the reaction mixture to contain at least the stoichiometric requirement of the acceptor, and preferably a stoichiometric excess, in order for the product to contain a substantial amount of a desired product molecule. Thus, e.g., when a product containing three acceptor moieties is desired, the reaction mixture should contain at least the stoichiometrically required three mols of acceptor/mol of donor and preferably contains >3 mols of acceptor/mol of donor; and, when a product containing eight acceptor moieties is desired, it is important for the reaction mixture to contain at least eight mols of acceptor/mol of donor.

Since the product molecules contain 1-30 acceptor moieties/donor moiety, the acceptor/donor mol ratio in the reaction mixture is most commonly 1-35/1, more preferably 1-10/1, and at least 3/1 when the novel compounds are desired.

The mixture of compounds of the invention, comprises the compounds corresponding to the formula $Z\text{-}C(E)(E')_p\text{-}Q_s$ in which Z is alkyl, cycloalkyl, or $\text{-(CTT'-CT''G)}_w\text{-CTT'-CHT''G}$; Q is $\text{-(CTT'-CT''G)}_t\text{-CTT'-CHT''G}$; E' is an electron withdrawing group; T, T', T'', E and G have the definitions given above; $p$ is zero or one; $s$ is respectively two or one; and each of $t$ and $w$ represents zero or a positive integer such that the compound contains at least three, preferably 3-30 G groups.

The most preferred of the $Z\text{-}C(E)(E')_p\text{-}Q_s$ compounds are (A) those in which $p$ and $s$ are one; Z is $\text{-(CTT'-CT''G)}_w\text{-CTT'-CHT''G}$; E, E', G, and G' are independently selected from -CN, -COOR, and -C(O)R' groups wherein R and R' represent aliphatic, cycloaliphatic, or alphyl groups containing up to 30 carbons, usually alkyl or cycloalkyl groups containing up to 10 carbons; and the sum of $t$ and $w$ is 1-30, preferably 1-10, and (B) those in which $s$ is two; Z is $\text{-(CTT'-CT''G)}_w\text{-CTT'-CHT'G}$; E, G, and G' are independently selected from -CN, -COOR, and -C(O)R' groups wherein R and R' represent aliphatic, cycloaliphatic, or alphyl groups containing up to 30 carbons, usually alkyl or cycloalkyl groups containing up to 10 carbons; and the sum of $t$ and $w$ is 0-30, preferably 1-10.

These compounds and the corresponding oils containing fewer than three acceptor moieties, e.g., the compounds having the above structures in which the sum of $t$ and $w$ is 0-3, are prepared from the aforementioned Z'-CH (E)(E'') donors and CTT'=CT''G acceptors as illustrated in the following equations:

$$\text{I.} \quad CH_3CH(COOCH_3)_2 + t + \mathit{1}\ CH_2\text{=}CHCOOCH_3 \rightarrow$$

$$CH_3C(COOCH_3)_2\text{-}[(CH_2CHCOOCH_3)_t\text{-}CH_2CH_2COOCH_3]$$

$$\text{II.} \quad CH_2(COOCH_3)_2 + t\text{+}2\ CH_2\text{=}CHCOOCH_3 \rightarrow$$

$$CH_3OOCCH_2CH_2\text{-}C(COOCH_3)_2\text{-}[(CH_2CHCOOCH_3)_t\text{-}CH_2CH_2COOCH_3]$$

III.    $CH_2(CN)_2 + t+w+2 \ CH_2=CHCN \rightarrow$

$[CNCH_2CH_2-(CNCHCH_2)_w]-C(CN)_2-[(CH_2CHCN)_t-CH_2CH_2CN]$

IV.    $CH_3C(O)CH_2COOCH_3 + t+2 \ CH_2=CHCOOCH_3 \rightarrow$

$CH_3C(O)C(COOCH_3)(CH_2CH_2COOCH_3)-[(CH_2CHCOOCH_3)_t-CH_2CH_2COOCH_3]$

These reactions are able to proceed until the desired number of acceptor moieties have been combined with the donor, even when the donor contains only one active hydrogen, because the hydrogen donated to an acceptor moiety when the donor is deprotonated becomes an active hydrogen in the acceptor moiety and can be donated to a second moiety where it becomes a donatable hydrogen again.

As indicated by Equations II and III above, the incorporation of several acceptor moieties into the product molecules is facilitated by utilizing the more reactive $CH_2(E)(E'')$ donors, especially when a $CH_2=CHG$ acceptor is employed; and the incorporation of multiple acceptor moieties is also aided by the use of (1) reactants containing the stronger electron withdrawing groups, (2) the higher reaction temperatures, (3) the stronger catalysts, and/or (4) the larger amounts of catalyst. Variations in product structure and properties can be achieved by using mixtures of donor compounds and/or mixtures of acceptor compounds in the reaction.

The products of the Michael reaction may be liquids or solids, depending on the particular reactants and reactant ratios used; and, as already indicated, they are typically mixtures of compounds containing different numbers of acceptor moieties per molecule. If desired, the individual compounds of the mixture or groups of those compounds may be separated from one another prior to being used in their end application or prior to being subjected to additional reactions preparatory to such use. Separations that are sometimes beneficial are (1) separations of individual compounds containing at least three acceptor moieties per molecule from one another and/or from the lower molecular weight products and (2) other separations of relatively low and relatively high molecular weight fractions. However, the product mixtures themselves — especially those in which at least about 25% of the product molecules contain at least three acceptor moieties — are also useful materials, so such separations are frequently unnecessary and, in fact, sometimes undesirable. Having a product characterized by a wide molecular weight distribution can be an advantage in providing a balance of properties, as is the case with oils which are to be used in an application wherein some relatively high molecular weight portion is desired to give a required viscosity, but some relatively low molecular weight portion is desired to impart compatibility with a material with which the oil is to be used.

Achieving either a better balance of properties or properties which differ in some other respect from those of the Michael reaction product can also be accomplished by subjecting the product mixture or one or more of the components thereof to one or more of the reactions known to be capable of converting functional groups (i.e., E, E', G, and/or G' groups) in the compounds to different groups. Such reactions, such as the conversion of lower ester groups to higher ester groups, can be conducted by conventional techniques, such as those indexed and outlined in Harrison and Harrison, *Compendium of Organic Synthetic Methods*, Wiley-Interscience (New York), 1971.

In addition to having the aforementioned advantage, the post-treatment of the Michael reaction product to prepare a different compound or product mixture of the invention has the benefit of facilitating the preparation of products which it would be at least more difficult to prepare directly by the Michael reaction. For example, it can be beneficial to use a post-Michael reaction conversion of the functional groups when the desired end product is to contain functional groups which, if present in the Michael-reactants, would make the Michael reaction relatively slow. Thus, it is apt to be preferred, for example, to react dimethyl malonate with methyl acrylate to provide a first product and then transesterify that product with hexanol to provide a second product in which the functional groups are hexyl ester groups than to prepare a Michael reaction product from the slower-reacting dihexyl malonate and hexyl acrylate.

Since the Michael reaction and the post-Michael reaction treatments of the Michael products can be tailored to form products which are liquids or solids having widely different molecular weights, the different products of the invention are useful in a variety of applications — the lower molecular weight products being generally most suitable as plasticizers and solvents, the oils usually serving best as lubricants, and the higher molecular weight solids ordinarily being most suited as plastics.

The $Z-C(E)(E')_p-Q_s$ oils, and most especially the ester oils, constitute a preferred embodiment of the invention. These oils, in addition to having general utility as lubricants, have particular value as refrigeration lubricants, since (1) they can be adapted to have viscosities suitable for refrigeration lubricants, and (2) their high polarity, together with extensive branching and molecular weight tailoring, can make them completely miscible with common refrigerants, e. g., ammonia; alcohols such as methanol and ethanol; glycols such as ethylene and propylene glycols; hydrocarbons such as methane, ethane, propane (R-290), butane, ethylene, and propylene; and halocarbons and/or halohydrocarbons such as chlorotrifluoromethane, dichlorodifluoromethane, dichlorofluoromethane, chlorodifluoromethane (R-22), 1,2,2-trifluoro-1,1,2-trichloroethane, 1,1-dichloro-2,2,2-trifluoroethane (R-123), 1,1-dichloro-1-fluoroethane, 1-chloro-2,2,2-trifluoroethane, 1-chloro-1,2,2,2-tetrafluoroethane (R-124), 1-chloro-1,1,2,2-tetrafluoroethane, dichloromethane, difluoromethane (R-32), 1,1,2,2,2-pentafluoroethane (R-125), 1,1,2,2-tetrafluoroethane (R-134), 1,1,1,2-tetrafluoroethane (R-134a), 1,1,1-trifluoroethane (R-143a), 1,1-difluoroethane (R-152a), and mixtures thereof.

Among the refrigerant blends with which these oils can be advantageously used as lubricants are the binary mixtures of R-32 with R-125, R-152a, or R-134a; R-125/R-143a, R-290/R-134a, and R-22/R-152a binary blends; and ternary blends such as R-22/R-290/R-125, R-22/R-152a/R-124, R-32/R-125/R-134a, and R-125/R-143a/R-134a.

The ability of the present invention to provide lubricants compatible with R-134a is perhaps its most valuable asset, since R-134a has been reported to have an ozone depletion potential of zero, therefore would be environmentally superior to the chlorofluorocarbon refrigerants most commonly used in refrigeration applications, but has the disadvantage of not being compatible with the lubricants normally used in those applications. However, as already mentioned, the ester oils have general utility as lubricants; and they have uses in other applications too. For example, (1) having relatively low volatilities at given viscosities, they can be utilized as hydraulic fluids in metal working, electrical generation, and mining industries, optionally in conjunction with soluble polymers, such as styrene-diene polymers, (2) having a relatively high smoke point for a given viscosity, they can be used in spin finish formulations in the textile industry and in other such applications where it is undesirable to release smoke into the working environment, and (3) those having the best thermal stabilities can also be employed in applications such as turbine oils, rolling oils, and compressor oils.

The ester oils are preferably prepared by (1) reacting a $Z'$-$CH(COOR)_2$ donor in which $Z'$ is most preferably hydrogen with a $CTT'$=$CT''COOR$ acceptor to form a $Z$-$C(COOR)_2$-$(CTT'$-$CT''COOR)_w$-$CTT'$-$CHT''COOR$ product composed primarily of molecules wherein $Z$ is most preferably -$(CTT'$-$CT''COOR)_t$-$CTT'$-$CHT''COOR$, at least some of the Rs (which may be the same or different) are lower alkyls of 1-8 carbons, each of $t$ and $w$ is zero or a positive integer, and the sum of $t$ and $w$ is 0-28 and (2) when desired (especially when all of the Rs are methyl) transesterifying the resultant intermediate product by reacting it with one or more alcohols containing more carbons per molecule than the lower alkyl groups of the intermediate. An especially preferred embodiment of the invention resides in preparing such ester oils having at least three acceptor moieties in at least about 25%, preferably at least about 40% of the molecules obtained by the Michael reaction.

In the practice of these preferred embodiments of the invention, the intermediate product may be recovered from its synthesis reaction mixture and, if desired, may also be fractionated into separate components before being subjected to transesterification. However, it is frequently preferable to transesterify the intermediate without first separating it from its synthesis reaction mixture.

Regardless of whether the transesterification is conducted on a recovered or unrecovered intermediate, it is accomplished by contacting the intermediate with one or more alcohols containing more carbons per molecule than the alkyl groups to be replaced and maintaining contact between the reactants at a suitable temperature until the desired transesterification has been effected. Alcohols most apt to be desirable for use in the reaction are substituted and unsubstituted alkanols, cycloalkanols, and aralkanols containing up to about 30 carbons (e.g., ethanol, chloroethanol, propanol, butanol, hexanol, bromohexanol, heptanol, octanol, decanol, fluorodecanol, dodecanol, hexadecanol, octadecanol, eicosanol, tetracosanol, triacontanol, cyclohexanol, cyclooctanol, benzyl alcohol, p-methylbenzyl alcohol, phenethyl alcohol, phenylpropanol, phenylpentanol, and phenethylbenzyl alcohol), as well as the aliphatic, cycloaliphatic, and araliphatic alcohols containing up to 30 carbons and also containing hetero atoms, such as oxygen, phosphorus, or sulfur (e.g., ethylthioethanol, ethoxyethanol, and the like).

The amount of alcohol employed in the transesterification reaction varies with the degree of transesterification desired, the quantity generally being the stoichiometric amount or an amount slightly in excess of the stoichiometric requirement. For example, when the intermediate contains an average of four ester groups per molecule, and it is wished to replace substantially all of those ester groups with the alcohol or alcohols used in the transesterification reaction, the amount of alcohol added to the intermediate should be at least four mols/mol of intermediate. Only about half as much alcohol would be added, on the other hand, when the objective is to replace approximately half of the ester groups of the intermediate.

Use of a transesterification reaction after completion of the Michael reaction permits a wide variety of products to be prepared from any particular product of the Michael reaction — final products having only the short ester chains which favor solubility in a refrigerant such as R-134a, final products having only the longer ester chains which increase viscosity, and final products having a controlled mix of short and longer ester chains to provide desired intermediate degrees of solubility and viscosity.

The transesterification is suitably conducted at an elevated temperature which provides for reflux and removal of a lower alcohol by-product from the reaction mixture without permitting undue loss of the higher alcohol reactant(s) from the reaction vessel, e.g, a temperature of 50-180°C. Although the reaction does not require catalysis, it is accelerated by the use of a base, which may be the base already present when the Michael reaction product is transesterified without first being recovered from its synthesis reaction mixture. It is sometimes desirable to add a catalytic amount of a base to accelerate the reaction, especially when the Michael product has been recovered before being subjected to transesterification. However, when such an addition is made, the amount of catalyst added is preferably kept low enough to prevent interference with the reaction or with subsequent separation of the products from the reaction mixture. Such an amount is typically 0.05-1.0 g/kg of the Michael reaction product to be transesterified.

In another preferred embodiment of the invention, reaction products obtained from Michael donors and acceptors in which less than all of the electron withdrawing groups are ester groups (e.g, products obtained from methyl acetoacetate and methyl acrylate and products obtained from dimethyl malonate and methacrylonitrile) are subjected to a transesterification reaction to replace some or all of the ester groups with higher ester groups. Desirable ester products can also be obtained by subjecting a Michael reaction product containing nitrile groups to simultaneous hydrolysis and esterification with one or more alcohols in order to replace some or all of the nitrile groups with ester groups.

The products resulting from the Michael reaction or from conversion of the Michael reaction products to derivatives are typically washed with water to remove any unreacted materials and catalyst prior to being used in their intended application; and, if desired, they may then be further purified by subjecting them to fractional distillation. They may then be utilized alone or together with other materials serving similar functions and/or with additives serving other functions in their intended application, e.g., as plasticizers, solvents, lubricants, molding materials, or any of the other uses mentioned above.

Additives particularly apt to be used together with the products of the invention are (1) the antioxidants frequently used in organic compositions, (2) epoxy and other dehydrating agents sometimes used in refrigeration compositions, and (3) the oxidation resistance and thermal stability improvers, corrosion inhibitors, metal deactivators, lubricity additives, viscosity index improvers, pour and/or floc point depressants, detergents, dispersants, antifoaming agents, anti-wear agents, and extreme pressure resistance additives conventionally used in lubricant compositions, e.g., the additives exemplified in U.S. Patent 5,021,179 (Zehler et al.).

When the oils are employed as refrigeration lubricants, they are used in conjunction with refrigerants (such as those mentioned above) to provide refrigeration compositions which typically comprise 0.001-1, preferably 0.1-1 part of lubricant per part by weight of the refrigerant— additives, such as those mentioned above, generally constituting only a minor amount of the lubricant (e.g., up to 8%, preferably not more than 5%, of the weight of the lubricant formulation) when utilized. The refrigeration compositions are ordinarily formed prior to use. However, when desired, they may also be formed in situ during operation of the refrigeration equipment. Thus, the refrigerant and the lubricant may be charged to the refrigeration equipment separately, either simultaneously or consecutively in either order, instead of being preblended.

In choosing an oil to use as a lubricant with a refrigerant, it is important to select one which is completely miscible with the refrigerant throughout the temperature range to which the refrigeration composition is to be exposed and which has a viscosity such as to permit its functioning as a lubricant over that entire temperature range. The optimum lubricant to be used in any instance can be determined by routine experimentation, aided by observation of the following general principles:

(1) Miscibility with refrigerants is enhanced by the presence of short side chains in the oil molecules.
(2) A low viscosity is most suitable for a lubricant to be used at relatively low temperatures, while lubricants intended for use at relatively high temperatures should have higher viscosities.
(3) Viscosity is increased by the presence of long side chains in the molecules as well as by having a larger number of side chains therein; and variations in viscosity may thus be achieved by varying the number of long-chain groups in the oils, increasing or decreasing their molecular weights, and/or widening or narrowing their molecular weight distributions.
(4) The viscosities most suitable for lubricants to be used in refrigeration compositions that are to be exposed to the temperature conditions generally found in refrigeration equipment (i.e., temperatures in the range of -40°C to 70°C or sometimes even higher temperatures) are apt to be 1-600, preferably 5-300, and most preferably 10-200 $mm^2 \cdot s^{-1}$ at 40 ° C; and it is frequently also desirable for the lubricant to have a viscosity index $\geq 100$.

As already indicated, a particularly valuable aspect of the present invention is its provision of oils capable of being used as lubricants in refrigeration compositions comprising fluorohydrocarbon refrigerants, such as R-134a. The lubricants of the invention include many which have sufficient miscibility with R-134a to be utilizable therewith. However, the lubricants which are apt to be preferred in this regard are the $ROOC-CH_2CH_2-(ROOC-CHCH_2)_w-C(COOR)_2-(CH_2CHCOOR)_t-CH_2CH_2COOR$ oily mixtures in which the R's represent one or more alkyl groups of 1-30 carbons and the sum of $t$ and $w$ in the molecules is an average of 0-10. Of these preferred lubricants, those which are most preferred are usually the mixtures in which all of the R groups are isopropyl or in which at least 10% of the R groups are methyl groups and at least 50% of the R groups are alkyl groups of 4-10 carbons.

The following examples are given to illustrate the invention and are not intended as a limitation thereof.

EXAMPLE 1

Reaction of dimethyl malonate with methyl acrylate

Charge a suitable reaction vessel with 792g (6 mols) of dimethyl malonate, 52.8g (0.4 mol) of potassium carbonate, 12g (0.035 mol) of tetrabutylammonium hydrogen sulfate, and 1290g (15 mols) of methyl acrylate. After stirring the reaction mixture at room temperature for ∼18 hours, slowly heat it to ∼50 ° C to effect a rapid rise of the temperature of the reaction mixture to reflux. Maintain the reaction mixture at reflux for ∼15 minutes and then cool to room temperature over a period of ∼1 hour. A heavy solid mass forms in the bottom of the reaction vessel during cooling. Dilute this mass with methylene chloride, wash with five 1.5-L portions of water, and subject the product to gas chromatographic (GC) analysis. The analysis shows the product to consist, in area percentages, of 4.3% trimethyl ester of 1,1,3-propanetricarboxylic acid, 70% tetramethyl ester of 1,3,3,5-pentanetetracarboxylic acid, 18% pentamethyl ester of 1,3,3,5,7-heptanepentacarboxylic acid, and 7.7% polyesters, i.e., products having more than five ester groups per molecule. Isolate the triester and tetraester components by fractional distillation under reduced pressure.

EXAMPLE 2

Reactions of dimethyl malonate with methyl acrylate

Conduct two additional Michael reactions between dimethyl malonate and methyl acrylate using tetrabutylammonium hydrogen sulfate as the phase transfer catalyst as in Example 1 but employing sodium methoxide as the base, 80°C as the reaction temperature, and methyl acrylate/dimethyl malonate mol ratios of 8/1 (reaction mixture 2-A) and 10/1 (reaction mixture 2-B) respectively. Monitor the reactions by GC and discontinue them when the following analyses are obtained:

Reaction mixture 2-A: 32% tetramethyl ester of 1,3,3,5-pentanetetracarboxylic acid, 24% pentamethyl ester of 1,3,3,5,7-heptanepentacarboxylic acid, 11% hexamethyl ester of 1,3,5,5,7,9-nonanehexacarboxylic acid, 8% heptamethyl ester of 1,3,5,5,7,9,11-undecaneheptacarboxylic acid, 2% octamethyl ester of 1,3,5,7,7,9,11,13-tridecaneoctacarboxylic acid, and smaller amounts of higher esters

Reaction mixture 2-B: 20% tetramethyl ester, 22% pentamethyl ester, 19% hexamethyl ester, 14% heptamethyl ester, 9% octamethyl ester, and smaller amounts of higher esters

Then work up the product mixtures by diluting them with solvent, washing to neutrality with water, and removing solvent, water, and lower boiling products by distillation to form viscous oils which, in each case, are completely miscible with R-134a over a temperature range of -40° C to 70 ° C.

EXAMPLE 3

Reaction of dimethyl malonate with butyl acrylate

Charge a suitable reaction vessel with 660g (5 mols) of dimethyl malonate, 35g (0.25 mol) of potassium carbonate, and 1.75g (0.005 mol) of tetrabutylammonium hydrogen sulfate. Heat the stirred mixture to 120°C, and add 2048g (16 mols) of n-butyl acrylate over a period of six hours while monitoring the reaction by GC, which shows the dibutyl dimethyl ester of 1,3,3,5-pentanetetracarboxylic acid to be the major product at the end of this period. Then heat the reaction mixture at 150°C for three hours to form a product mixture containing the tributyl dimethyl ester of 1,3,3,5,7-heptanepentacarboxylic acid. Cool the resulting reaction mixture to room temperature, add water and toluene, wash repeatedly with water until neutral, remove the water and toluene by azeotropic distillation, and then remove light products at 180-185 ° C and 0.1-0.15 mmHg to provide a heavy oil having a viscosity of 96 mm$^2$·s$^{-1}$ at 40 ° C, a viscosity of 11.6 mm$^2$·s$^{-1}$ at 100 ° C, a viscosity index of 109, and excellent miscibility with R-134a over a temperature range of -60 ° C to 80 ° C.

EXAMPLE 4

Transesterification of mixed esters with alcohol mixture

Using a dimethyl malonate/methyl acrylate Michael reaction and workup procedure similar to that of the preceding examples, prepare a 20.8g sample of a mixture of 66% tetramethyl ester of 1,3,3,5-pentanetetracarboxylic acid, 26% pentamethyl ester of 1,3,3,5,7-heptanepentacarboxylic acid, and 6% hexamethyl and heptamethyl esters. Treat the mixture with 0.1 mol of butanol and 0.1 mol of hexanol at 120°C in the presence of a catalytic amount of 10% sodium methoxide, remove the volatiles by distillation, and work up to provide an oil which has a viscosity of 159 mm$^2$·s$^{-1}$ at 40°C, a viscosity of 14.6 mm$^2$·s$^{-1}$ at 100°C, a viscosity index of 88, and total miscibility with R-134a.

EXAMPLE 5

Transesterification of tetramethyl ester with alcohol mixture

Charge a suitable reaction vessel with 660g (2.2 mols) of the tetramethyl ester of Example 1, 406g (4.4 mols) of n-butanol, 560g (4.4 mols) of n-hexanol, and 5 mL of 5% sodium methoxide. Stir the reaction mixture magnetically and heat to ~ 110° C to result in the slow distillation of methanol. After removing a stoichiometric amount of methanol, cool the reaction mixture to room temperature and dilute with toluene. After washing with water, remove the solvent and distill the crude oil under reduced pressure. The fraction collected at 195-220 ° C and 0.11-0.14 mm Hg is a water-white oil containing the tetraester product. This oil is miscible with R-134a refrigerant over a temperature range of -40 ° C to 70°C and has a viscosity of 20-30 mm$^2$·s$^{-1}$ at 40 ° C, a viscosity index of 100, and a total acid number (TAN) of <0.05 mg KOH/gram.

EXAMPLE 6

Transesterification of 1.5-dibutyl-3.3-dimethyl ester with 2-ethylhexanol

Transesterify a crude reaction mixture of 85% 1,5-dibutyl-3,3-dimethyl ester of 1,3,3,5-pentanetetracarboxylic acid with 21g (0.16 mol) of 2-ethyl-1-hexanol at 150-200 ° C under nitrogen. After removing the stoichiometric amount of methanol, cool the reaction mixture, dilute with toluene, wash to neutrality with water, and remove the water and toluene by azeotropic distillation. The resultant oil has a viscosity of 62.9 mm$^2$·s$^{-1}$ at 40° C, a viscosity of 7.9 mm$^2$·s$^{-1}$ at 100°C, a viscosity index of 88, and total miscibility with R-134a.

EXAMPLE 7

Transesterification of 3,3-diethyl-1,5-dimethyl ester with butanol

Transesterify a 3,3-diethyl-1,5-dimethyl ester of 1,3,3,5-pentanetetracarboxylic acid with n-butanol in the presence of a catalytic amount of sodium methoxide by the general procedure of Example 5. The resulting product is totally miscible with R-134a refrigerant, has a viscosity of 19.2 mm$^2$·s$^{-1}$ at 40°C, a viscosity of 3.6 mm$^2$·s$^{-1}$ at 100°C, and a viscosity index of 40.

EXAMPLE 8

Transesterification of 3,3-diethyl-1,5-dimethyl ester with alcohol mixture

Repeat Example 7 except for replacing the butanol with a 1/1/1 mixture of n-hexanol, n-heptanol, and n-octanol. The resulting product is totally miscible with R-134a refrigerant at temperatures of 0-70°C, has a viscosity of 22.3 mm$^2$·s$^{-1}$ at 40°C, a viscosity of 4.4 mm$^2$·s$^{-1}$ at 100 ° C, and a viscosity index of 107.

EXAMPLE 9

One-pot Michael addition and transesterification

Charge a reaction vessel with 15.8 Kg (120 mols) of dimethyl malonate, 158g (1.2 mols) of potassium carbonate, and 37g (0.1 mol) of tetrabutylammonium hydrogen sulfate under nitrogen. Heat the reactor to ~70 ° C, add 25.8 Kg (300 mols) of methyl acrylate over six hours, and then heat the reaction mixture at 70-80 ° C for at least 10 hours to form a product mixture containing a major amount of tetramethyl ester of 1,3,3,5-pentanetetracarboxylic acid, smaller amounts of pentamethyl and higher esters, and a minor amount of trimethyl ester of 1,1,3-propanetricarboxylic acid.
Charge 22 Kg (296 mols) of n-butanol and 30.3 Kg (296 mols) of n-hexanol to the reactor and heat at 110-120°C while collecting the volatiles overhead. After removing the stoichiometric amount of methanol, cool the reaction mixture to room temperature, dilute with toluene, wash to neutrality with water, dry by the azeotropic removal of water, and heat treat the crude under reduced pressure.

Distillation under reduced pressure (1 mmHg) and 200-250 ° C provides an oil which has a viscosity of 17 $mm^2 \cdot s^{-1}$ at 40 °C, a viscosity of 3.6 $mm^2 \cdot s^{-1}$ at 100°C, a total acid number (TAN) of 0.025 mgKOH/g, a water content of 64 ppm, and total miscibility with R-134a over a temperature range of -60°C to 80°C. The bottoms product is an oil having a viscosity of 24.8 $mm^2 \cdot s^{-1}$ at 40°C, a viscosity of 4.7 $mm^2 \cdot s^{-1}$ at 100°C, a total acid number of 0.034 mgKOH/ g, a water content of 73 ppm, and total miscibility with R-134a over a temperature range of -60°C to 80°C.

EXAMPLE 10

Reaction of malononitrile with acrylonitrile

Charge a reaction vessel with 3.3g (0.05 mol) of malononitrile, 0.7g (0.005 mol) of potassium carbonate, and 0.17g (0.5 mmol) of tetrabutylammonium hydrogen sulfate under nitrogen. Slowly add 11.2g (0.2 mol) of acrylonitrile at 50°C with stirring and maintain the temperature at 50-70 ° C for 3 hours. Then cool the reaction mixture to room temperature, dissolve in ethyl acetate, wash with water until neutral, dry over magnesium sulfate, filter, and concentrate to provide a solid mass which spectroscopic analysis indicates to contain more than two acrylonitrile moieties per molecule.

EXAMPLE 11

Reaction of malononitrile with methyl acrylate

Charge a reaction vessel with 3.3g (0.05 mol) of malononitrile, 0.7g (0.005 mol) of potassium carbonate, and 0.17g (0.5 mmol) of tetrabutylammonium hydrogen sulfate. Heat the mixture to 50° C under nitrogen and slowly add 10.8g (0.125 mol) of methyl acrylate at a rate such as to maintain the temperature under 80 ° C. Keep the reaction mixture at 70-80 ° C for two hours, cool to room temperature, dilute with dichloromethane, wash with water until neutral, dry over magnesium sulfate, filter, and concentrate to provide a solid mass which spectroscopic analysis shows to contain dimethyl ester of 3,3-dicyano-1,5-pentanedicarboxylic acid, trimethyl ester of 5,5-dicyano-1,3,7-heptanetricarboxylic acid, and smaller amounts of higher molecular weight components.

**Claims**

1. A mixture of compounds corresponding to the formula $Z-C(E)(E')_p-Q_s$ in which Z is alkyl, cycloalkyl, or -(CTT'-CT"G)$_w$-CTT'-CHT"G; Q is -(CTT'-CT"G)$_t$-CTT'-CHT"G; T, T', and T" are independently selected from hydrogen, G', and alkyl, alkoxyalkyl, alkylthioalkyl, cycloalkyl, dialkylaminocycloalkyl, aryl, haloaryl, alkoxyaryl, aralkyl, and alkaryl organic groups containing up to 20 carbons; E, E', G, and G' are independently selected from -COOR, -C(O)R', and -CN electron withdrawing groups in which R and R' represent alkyl or cycloalkyl groups of up to 10 carbons; $p$ is zero or one; $s$ is respectively two or one; and each of $t$ and $w$ represents zero or a positive integer; each compound in the mixture containing 1-30 G groups per E group, at least two compounds in the mixture containing a different number of G groups, and at least 25% of the molecules of the compounds containing at least three G groups.

2. The mixture of claim 1 wherein $p$ and $s$ are one; Z is -(OTT'-CT"G)$_w$-CTT'-CHT"G; and the sum of $t$ and $w$ in at least 25% of the molecules is 1-30.

3. The mixture of claim 2 wherein T, T', and T" are independently selected from hydrogen, G', and hydrocarbyl or predominantly hydrocarbyl groups containing up to 20 carbons, with the proviso that at least one of T, T', and T" is hydrogen.

4. The mixture of claim 3 wherein at least some of the electron withdrawing groups are -COOR groups.

5. The mixture of claim 4 wherein Z is -(CH$_2$CHCOOR)$_w$-CH$_2$CH$_2$COOR; Q is -(CH$_2$CHCOOR)$_t$-CH$_2$CH$_2$COOR; E and E' are -COOR groups; and the -COOR groups of Z, Q, E, and E' are independently selected from -COOR groups in which R is an alkyl of 1-10 carbons.

6. The mixture of claim 5 wherein at least 10% of the R groups are methyl groups, at least 50% of the R groups are alkyl groups of 4-10 carbons, and the sum of $t$ and $w$ in at least 25% of the molecules is 1-10.

**7.** The mixture of any of the preceding claims which is an oil.

**8.** A process for preparing a mixture of any of claims 1-7 which comprises reacting one molar proportion of at least one Michael donor corresponding to the formula Z'-CH(E)(E") wherein Z' is hydrogen, alkyl, or cycloalkyl; E is an electron withdrawing group, and E" is hydrogen or an electron withdrawing group with 3-35 molar proportions of at least one Michael acceptor corresponding to the formula CTT'=CT"G in the presence of a basic compound and a phase transfer catalyst until at least 25% of the product molecules contain at least three acceptor moieties.

**9.** The process of claim 8 which comprises reacting at least one Michael donor corresponding to the formula $CH_2(COOR)_2$ with at least one Michael acceptor corresponding to the formula $CH_2=CHCOOR$ until at least 25% of the product molecules correspond to the formula $ROOCCH_2CH_2\text{-}(ROOCCHCH_2)_w\text{-}C(COOR)_2\text{-}(CH_2CHCOOR)_t\text{-}CH_2CH_2COOR$ in which the sum of $t$ and $w$ is 1-28 and the -COOR groups are independently selected from -COOR groups in which R is an alkyl of 1-8 carbons.

**10.** The process of claim 9 wherein the product of the reaction between the Michael donor and Michael acceptor is reacted with one or more alcohols containing more carbons than at least some alkyls of the Michael reaction product to replace at least some of those alkyls with higher hydrocarbyl groups containing up to 30 carbons.

**11.** A refrigeration composition comprising a refrigerant and, as a refrigeration lubricant, a mixture of claim 7.

**12.** The composition of claim 11 wherein the refrigerant comprises at least one fluorohydrocarbon.

**13.** The composition of claim 12 wherein the refrigerant is 1,1,1,2-tetrafluoroethane.

## Patentansprüche

**1.** Gemisch von Verbindungen der Formel $Z\text{-}C(E) (E')_p\text{-}Q_s$, worin Z für Alkyl, Cycloalkyl oder $\text{-}(CTT'\text{-}CT"G)_w\text{-}CTT'\text{-}CHT"G$ steht, $Q\text{-}(CTT'\text{-}CT"G)_t\text{-}CTT'\text{-}CHT"G$ ist, T, T' und T" unabhängig voneinander aus Wasserstoff, G' und organischen Alkyl-, Alkoxyalkyl-, Alkylthioalkyl-, Cycloalkyl-, Dialkylaminocycloalkyl-, Aryl-, Halogenaryl-, Alkoxyaryl-, Aralkyl- und Alkarylgruppen mit bis zu 20 Kohlenstoffatomen ausgewählt sind, E, E', G und G' unabhängig voneinander aus elektronenentziehenden -COOR, -C(O)R' und -CN-Gruppen ausgewählt sind, in denen R und R' Alkyl- oder Cycloalkylgruppen mit bis zu 10 Kohlenstoffatomen bedeuten; p 0 oder 1 ist, s 2 bzw. 1 ist und jedes t und w für 0 oder eine positive ganze Zahl steht, wobei jede Verbindung im Gemisch 1 bis 30 G-Gruppen pro E-Gruppe aufweist, mindestens zwei Verbindungen im Gemisch eine unterschiedliche Anzahl von G-Gruppen aufweisen und mindestens 25 % der Moleküle der Verbindungen mindestens drei G-Gruppen enthalten.

**2.** Gemisch nach Anspruch 1, in dem p und s 1 sind, Z $\text{-}(CTT'\text{-}CT"G)_w\text{-}CTT'\text{-}CHT"G$ ist und die Summe von t und w in mindestens 25 % der Moleküle 1 bis 30 ist.

**3.** Gemisch nach Anspruch 2, in dem T, T' und T" unabhängig voneinander aus Wasserstoff, G' und Hydrocarbylgruppen oder überwiegend aus Hydrocarbyl bestehenden Gruppen mit bis zu 20 Kohlenstoffatomen ausgewählt werden mit der Maßgabe, dass mindesten eines von T, T' und T" Wasserstoff ist.

**4.** Gemisch nach Anspruch 3, in dem mindestens einige der elektronenentziehenden Gruppen -COOR-Gruppen sind.

**5.** Gemisch nach Anspruch 4, in dem Z $\text{-}(CH_2CHCOOR)_w\text{-}CH_2CH_2COOR$ ist; Q $\text{-}(CH_2CHCOOR)_t\text{-}CH_2CH_2COOR$ ist, E und E' -COOR-Gruppen sind und die -COOR-Gruppen von Z, Q, E und E' unabhängig voneinander aus -COOR-Gruppen ausgewählt werden, in denen R ein Alkyl mit 1 bis 10 Kohlenstoffatomen ist.

**6.** Gemisch nach Anspruch 5, in dem mindestens 10 % der R-Gruppen Methylgruppen und mindestens 50 % der R-Gruppen Alkylgruppen mit 4 bis 10 Kohlenstoffatomen sind und die Summe von t und w in mindestens 25 % der Moleküle 1 bis 10 ist.

**7.** Gemisch nach einem der vorstehenden Ansprüche, bei dem es sich um ein Öl handelt.

**8.** Verfahren zur Herstellung eines Gemischs nach einem der Ansprüche 1 bis 7, umfassend die Umsetzung eines Molanteils mindestens eines Michael-Donors der Formel Z'-CH(E)(E"), in der Z' Wasserstoff, Alkyl oder Cycloalkyl

**EP 0 618 956 B1**

ist, E eine elektronenentziehende Gruppe ist und E" Wasserstoff oder eine elektronenentziehende Gruppe ist, mit 3 bis 35 Molanteilen mindestens eines Michael-Akzeptors der Formel CTT'=CT"G in Gegenwart einer basischen Verbindung und eines Phasen-Transfer-Katalysators, bis mindestens 25 % der Produktmoleküle mindestens drei Akzeptorkomponenten aufweisen.

9. Verfahren von Anspruch 8, umfassend die Umsetzung mindestens eines Michael-Donors der Formel $CH_2(COOR)_2$ mit mindestens einem Michael-Akzeptor der Formel $CH_2=CHCOOR$, bis mindestens 25 % der Produktmoleküle der Formel $ROOCCH_2CH_2-(ROOCCHCH_2)_w-C(COOR)_2-(CH_2CHCOOR)_t-CH_2CH_2COOR$ entsprechen, in der die Summe von t und w 1 bis 28 ist und die -COOR-Gruppen unabhängig voneinander aus -COOR-Gruppen ausgewählt werden, in denen R ein Alkyl mit 1 bis 8 Kohlenstoffatomen ist.

10. Verfahren nach Anspruch 9, bei dem das Produkt der Reaktion zwischen dem Michael-Donor und dem Michael-Akzeptor mit einem oder mehreren Alkoholen, die mehr Kohlenstoffatome als mindestens einige Alkyle des Michael-Reaktionsprodukts enthalten, umgesetzt wird, um mindestens einen Teil dieser Alkyle durch höhere Hydrocarbylgruppen mit bis zu 30 Kohlenstoffatomen zu ersetzen.

11. Kühlzusammensetzung, die ein Kühlmittel und als Kühlschmiermittel ein Gemisch nach Anspruch 7 umfasst.

12. Zusammensetzung nach Anspruch 11, in der das Kühlmittel mindestens einen Fluorkohlenwasserstoff umfasst.

13. Zusammensetzung nach Anspruch 12, in der das Kühlmittel 1,1,1,2-Tetrafluorethan ist.


**Revendications**

1. Mélange de composés correspondant à la formule $Z-C(E)(E')_p-Q_s$ dans laquelle Z représente un groupe alkyle, cycloalkyle ou $-(CTT'-CT"G)_w-CTT'-CHT"G$; Q représente un groupe $-(OTT'-CT"G)_t-CTT'-OHT"G$ ; T, T' et T" sont choisis indépendamment entre l'hydrogène, G' et des groupes organiques alkyle, alkoxyalkyle, alkylthioalkyle, cycloalkyle, dialkylaminocycloalkyle, aryle, halogénaryle, alkoxyaryle, aralkyle et alkaryle contenant jusqu'à 20 atomes de carbone ; E, E', G et G' sont choisis indépendamment entre des groupes électrophiles -COOR, -C(O)R' et -CN dans lesquels R et R' représentent des groupes alkyle ou cycloalkyle ayant jusqu'à 10 atomes de carbone ; p est égal à zéro ou un ; s est, respectivement, égal à deux ou un ; et chacun des indices t et w est égal à zéro ou à un nombre entier positif ; chaque composé dans le mélange contenant 1 à 30 groupes G par groupe E, au moins deux composés dans le mélange contenant un nombre différent de groupes G, et au moins 25 % des molécules des composés contenant au moins trois groupes G.

2. Mélange suivant la revendication 1, dans lequel p et s sont égaux à un ; Z représente un groupe $-(CTT'-CT"G)_w-CTT'-CHT"G$ et la somme de t et w dans au moins 25 % des molécules est égale à une valeur de 1 à 30.

3. Mélange suivant la revendication 2, dans lequel T, T' et T" sont choisis indépendamment entre l'hydrogène, G' et des groupes hydrocarbyle ou principalement hydrocarbyle contenant jusqu'à 20 atomes de carbone, sous réserve qu'au moins un des groupes T, T' et T" représente l'hydrogène.

4. Mélange suivant la revendication 3, dans lequel au moins certains des groupes électrophiles sont des groupes -COOR.

5. Mélange suivant la revendication 4, dans lequel Z représente un groupe $-(CH_2CHCOOR)_w-CH_2CH_2COOR$ ; Q représente un groupe $-(CH_2CHCOOR)_t-CH_2CH_2COOR$ ; E et E' représentent des groupes -COOR ; et les groupes -COOR de Z, Q, E et E' sont choisis indépendamment parmi des groupes -COOR dans lesquels R représente un groupe alkyle ayant 1 à 10 atomes de carbone.

6. Mélange suivant la revendication 5, dans lequel au moins 10 % des groupes R sont des groupes méthyle, au moins 50 % des groupes R sont des groupes alkyle ayant 4 à 10 atomes de carbone et la somme de t et w dans au moins 25 % des molécules est égale à une valeur de 1 à 10.

7. Mélange suivant l'une quelconque des revendications précédentes, qui est une huile.

8. Procédé pour la préparation d'un mélange suivant l'une quelconque des revendications 1 à 7, qui comprend la réaction d'une proportion molaire d'au moins un donneur de Michael répondant à la formule Z'-CH(E)(E") dans laquelle Z' représente l'hydrogène, un groupe alkyle ou cycloalkyle ; E représente un groupe électrophile et E" représente l'hydrogène ou un groupe électrophile, avec 3 à 35 proportions molaires d'au moins un accepteur de Michael répondant à la formule CTT'=CT"G en présence d'un composé basique et d'un catalyseur de transfert de phase jusqu'à ce qu'au moins 25 % des molécules du produit contiennent au moins trois groupements accepteurs.

9. Procédé suivant la revendication 8, qui comprend la réaction d'au moins un donneur de Michael répondant à la formule $CH_2(COOR)_2$ avec au moins un accepteur de Michael répondant à la formule $CH_2=CHCOOR$ jusqu'à ce qu'au moins 25 % des molécules du produit répondent à la formule $ROOCCH_2CH_2-(ROOCCHCH_2)_w-C(COOR)_2-(CH_2CHCOOR)_t-CH_2CH_2COOR$ dans laquelle la somme de t et w est égale à une valeur de 1 à 28 et les groupes -COOR sont choisis indépendamment parmi des groupes -COOR dans lesquels R représente un groupe alkyle ayant 1 à 8 atomes de carbone.

10. Procédé suivant la revendication 9, dans lequel le produit de la réaction entre le donneur de Michael et l'accepteur de Michael est amené à réagir avec un ou plusieurs alcools contenant un plus grand nombre d'atomes de carbone qu'au moins certains groupes alkyle du produit de réaction de Michael pour remplacer au moins certains de ces groupes alkyle par des groupes hydrocarbyle supérieurs contenant jusqu'à 30 atomes de carbone.

11. Composition de réfrigération comprenant- un réfrigérant et, comme lubrifiant de réfrigération, un mélange suivant la revendication 7.

12. Composition suivant la revendication 11, dans laquelle le réfrigérant comprend au moins un fluoro-hydrocarbone.

13. Composition suivant la revendication 12, dans laquelle le réfrigérant est le 1,1,1,2-tétrafluoréthane.